# EUROPEAN PATENT SPECIFICATION

(11) **EP 4 396 773 B1**
(45) Date of publication and mention of the grant of the patent: **02.12.2026**
(21) Application number: 22777276.1
(22) Date of filing: 31.08.2022
(51) Int. Cl.: G06T 7/10

(54) **MANDIBULAR CANAL SEGMENTATION POSTPROCESSING**
NACHBEARBEITUNG DER SEGMENTIERUNG DES MANDIBULÄREN KANALS
POST-TRAITEMENT DE SEGMENTATION DE CANAL MANDIBULAIRE

(30) Priority: 01.09.2021 FI 20215916
(43) Date of publication of application: 10.07.2024
(73) Proprietor: Planmeca OY, 00880 Helsinki (FI)
(72) Inventor: LIN, Ziyuan, 00880 Helsinki (FI)
(86) International application number: PCT/FI2022/050573
(87) International publication number: WO 2023/031520

(56) References cited:
- XP093000649
- JASKARI JOEL ET AL: "Deep Learning Method for Mandibular Canal Segmentation in Dental Cone Beam Computed Tomography Volumes", SCIENTIFIC REPORTS, vol. 10, no. 5842, 3 April 2020 (2020-04-03), XP093000587, Retrieved from the Internet <URL:https://www.nature.com/articles/s41598-020-62321-3.pdf> [retrieved on 20221122], DOI: 10.1038/s41598-020-62321-3
- KEATMANEE CHADAPORN ET AL: "Inferior alveolar canal segmentation in cone beam computed tomography images using an adaptive diffusion flow active contour model", 2015 14TH IAPR INTERNATIONAL CONFERENCE ON MACHINE VISION APPLICATIONS (MVA), MVA ORGANIZATION, 18 May 2015 (2015-05-18), pages 57 - 60, XP033173121, DOI: 10.1109/MVA.2015.7153132
- ABDOLALI FATEMEH ET AL: "Automatic segmentation of mandibular canal in cone beam CT images using conditional statistical shape model and fast marching", INTERNATIONAL JOURNAL OF COMPUTER ASSISTED RADIOLOGY AND SURGERY, SPRINGER, DE, vol. 12, no. 4, 21 September 2016 (2016-09-21), pages 581 - 593, XP036195796, ISSN: 1861-6410, [retrieved on 20160921], DOI: 10.1007/S11548-016-1484-2
- KONDO T ET AL: "Computer-based extraction of the inferior alveolar nerve canal in 3-D space", COMPUTER METHODS AND PROGRAMS IN BIOMEDICINE, ELSEVIER, AMSTERDAM, NL, vol. 76, no. 3, 1 December 2004 (2004-12-01), pages 181 - 191, XP004610531, ISSN: 0169-2607, DOI: 10.1016/J.CMPB.2004.06.003
- GYEHYUN KIM ET AL: "Automatic Extraction of Inferior Alveolar Nerve Canal Using Feature-Enhancing Panoramic Volume Rendering", IEEE TRANSACTIONS ON BIOMEDICAL ENGINEERING, IEEE, USA, vol. 58, no. 2, 1 February 2011 (2011-02-01), pages 253 - 264, XP011343374, ISSN: 0018-9294, DOI: 10.1109/TBME.2010.2089053
- BRECHT MORIS ET AL: "Automated tracking of the mandibular canal in CBCT images using matching and multiple hypotheses methods", COMMUNICATIONS AND ELECTRONICS (ICCE), 2012 FOURTH INTERNATIONAL CONFERENCE ON, IEEE, 1 August 2012 (2012-08-01), pages 327 - 332, XP032245545, ISBN: 978-1-4673-2492-2, DOI: 10.1109/CCE.2012.6315922

## Description

### TECHNICAL FIELD

The present disclosure relates to dental imaging, and more particularly to a method for postprocessing of mandibular canal segmentation data, a corresponding computing device, and a computer program product.

### BACKGROUND

The human mandible, also known as the lower jaw, is anatomically complex and it is the only movable bone in the facial area facilitating the functions of mastication, speech and facial expressions. It also serves as a scaffold and platform for the lower dentition, muscle insertions, temporomandibular joint, nerves, and vessels. Important mandibular structures are the two mandibular canals that are located bilaterally underneath the teeth in the premolar and molar regions. Each canal contains the artery and vein as well as the inferior alveolar nerve, which is part of the mandibular branch of the trigeminus nerve that supplies the motor innervations to muscles and sensory innervations to the teeth, chin, and lower lip. Accurate localisation of mandibular canals in the lower jaws is important in dental implantology.

Non-Patent Literature "Deep Learning Method for Mandibular Canal Segmentation in Dental Cone Beam Computed Tomography Volumes" by JASKARI JOEL ET. AL., in SCIENTIFIC REPORTS, vol. 10, no. 5842, 3 April 2020, dicloses a deep learning system for automatic localisation of the mandibular canals, which includes a postprocessing method to refine the model predictions. As the model output has large quantities of false positives, likely due to sampling the training patches close to the mandibular canal, the output is filtered with a connected-component algorithm that connects neighbouring voxels to larger structures, from which the two largest structures are chosen as the predicted canals.

### SUMMARY

This summary is provided to introduce a selection of concepts in a simplified form that are further described below in the detailed description. This summary is not intended to identify key features or essential features of the claimed subject matter, nor is it intended to be used to limit the scope of the claimed subject matter.

It is an objective to provide a method for postprocessing of mandibular canal segmentation data. The foregoing and other objectives are achieved by the features of the independent claims. Further implementation forms are apparent from the dependent claims, the description and the figures.

According to a first aspect, a method for postprocessing of mandibular canal segmentation data comprises: obtaining mandibular canal segmentation data comprising a plurality of possible mandibular canal voxels in a computerized tomography scan of a mandible, wherein each voxel in the plurality of possible mandibular canal voxels is associated with a probability value quantifying a probability that the voxel comprises a mandibular canal; forming a plurality of voxel structures by connecting neighbouring voxels with a probability value above a preconfigured probability value threshold in the possible mandibular canal voxels; forming a plurality of routes by skeletonizing each voxel structure in the plurality of voxel structures, wherein each route in the plurality of routes comprises a spatial curve of a corresponding voxel structure in the plurality of voxel structures; inspecting, for each route pair in the plurality of routes, whether the route pair fulfils a concatenation criterion, and in response to a route pair fulfilling the concatenation criterion, concatenating the route pair, thus forming a second plurality of routes; and performing at least one check on at least one spatial feature of each route in the second plurality of routes and choosing at least one candidate mandibular canal pair from the second plurality of routes based on the at least one check. The method can, for example, provide the at least one candidate mandibular canal pair with improved accuracy.

In an implementation form of the first aspect, the method further comprises, after forming the plurality of routes and before inspecting, for each route pair in the plurality of routes, whether the route pair fulfils a concatenation criterion, sorting voxels in each route in the plurality of routes according to a relative location of each voxel in the route. The method can, for example, perform other operations more efficiently when the routes are sorted and thus provide the at least one candidate mandibular canal pair with improved efficiency.

In another implementation form of the first aspect, the performing of the at least one check on the at least one spatial feature of each route in the second plurality of routes and choosing at least one candidate mandibular canal pair from the second plurality of routes based on the at least one check comprises: determining a direction for a first segment of the route; for each segment in a plurality of segments of the route, other than the first segment of the route, inspecting whether a direction of the segment is within a preconfigured tolerance from the direction of the first segment of the route; calculating a monotonicity score based on a number of segments in the plurality of segments not being within the tolerance; and choosing the at least one candidate mandibular canal pair from the second plurality of routes based on at least the monotonicity score. The method can, for example, utilise directional information of the routes when choosing candidate mandibular canal pair and thus provide the at least one candidate mandibular canal pair with further improved accuracy.

In another implementation form of the first aspect, the performing of the at least one check on at least one spatial feature of each route in the second plurality of routes and choosing at least one candidate mandibular canal pair from the second plurality of routes based on the at least one check comprises: for at least one route pair in the second plurality of routes, calculating at least one pair score, wherein the at least one pair score comprises at least one of: a mean coordinate score for the route pair, wherein the mean coordinate score is calculated by comparing a mean coordinate in a coordinate axis direction of each route in the route pair, a symmetry score for the route pair quantifying spatial symmetry of the route pair; and/or a symmetry plane score for the route pair, calculating a total score for the at least one route pair in the second plurality of routes based on the at least one pair score of the pair; and choosing the at least one candidate mandibular canal pair from the second plurality of routes based on at least the total score. The method can, for example, utilise symmetry information of the route pairs when choosing candidate mandibular canal pair and thus provide the at least one candidate mandibular canal pair with further improved accuracy.

In another implementation form of the first aspect, the performing of the at least one check on the at least one spatial feature of each route in the second plurality of routes and choosing at least one candidate mandibular canal pair from the second plurality of routes based on the at least one check comprises: choosing a subset of routes from the second plurality of routes based on the monotonicity score of each route; calculating the at least one pair score for each route pair in the chosen subset of routes; calculating the total score for each route pair in the chosen subset based on the at least one pair score of the route pair; and choosing a route pair with a highest total score in the chosen subset of routes as the candidate mandibular canal pair. The method can, for example, utilise directional information of the routes and symmetry information of the route pairs when choosing candidate mandibular canal pair and thus provide the at least one candidate mandibular canal pair with further improved accuracy.

In another implementation form of the first aspect, the performing of the at least one check on the at least one spatial feature of each route in the second plurality of routes and choosing at least one candidate mandibular canal pair from the second plurality of routes based on the at least one check comprises: choosing a subset of routes from the second plurality of routes based on the monotonicity score of each route that quantifies how well its direction in general aligns with the direction of a real mandibular canal, in terms of spatial monotonicity; calculating the at least one pair score for each route pair in the chosen subset of routes; calculating the total score for each route pair in the chosen subset based on the at least one pair score of the route pair; and choosing a route pair with a highest total score in the chosen subset of routes as the candidate mandibular canal pair. The method can, for example, utilise directional information of the routes and symmetry information of the route pairs when choosing candidate mandibular canal pair and thus provide the at least one candidate mandibular canal pair with further improved accuracy.

In another implementation form of the first aspect, the coordinate axis direction is parallel with a width direction of the computerized tomography scan of the mandible. The method can, for example, utilise width (left-right) symmetry information of the routes when choosing candidate mandibular canal pair and thus provide the at least one candidate mandibular canal pair with further improved accuracy.

In another implementation form of the first aspect, the symmetry score and/or the symmetry plane score is calculated based on digests of the routes in the route pair, wherein the digest of a route comprises a location of a start of the route, a location of an end of the route, and a mean location of the route. The method can, for example, efficiently utilise the symmetry information of the routes and thus provide the at least one candidate mandibular canal pair with further improved accuracy and efficiency.

In another implementation form of the first aspect, the symmetry plane score quantifies how well a symmetry plane of the route pair is aligned with a height direction of the computerized tomography scan of the mandible. The method can, for example, utilise the symmetry plane information of the routes and thus provide the at least one candidate mandibular canal pair with further improved accuracy.

In another implementation form of the first aspect, the concatenation criterion comprises a distance between an endpoint of a first route in the route pair and an endpoint of a second route in the route pair being below a preconfigured maximum threshold distance. The method can, for example, efficiently determine whether the two routes should be concatenated and thus provide the at least one candidate mandibular canal pair with further improved accuracy and efficiency.

In another implementation form of the first aspect, the inspecting, for each route pair in the plurality of routes, whether the route pair fulfils the concatenation criterion comprises calculating at least one of: a minimum distance between endpoints of the route pair; a minimum distance between extrapolated endpoints of the route pair, wherein the extrapolated endpoints are obtained by linearly extrapolating the endpoints by a preconfigured number of voxels, and/or a skew distance of the route pair; wherein the concatenation criterion comprises the minimum distance between the endpoints fulfilling a first criterion, the minimum distance between the extrapolated endpoints fulfilling a second criterion, and/or the skew distance of the route pair fulfilling a third criterion. The method can, for example, determine whether the two routes should be concatenated based on these criteria and thus provide the at least one candidate mandibular canal pair with further improved accuracy.

In another implementation form of the first aspect, each voxel structure in the plurality of voxel structures is formed using a connected-component algorithm. The method can, for example, efficiently form the voxel structures using the connected-component algorithm and further utilise the properties of connected components and thus provide the at least one candidate mandibular canal pair with further improved accuracy and efficiency.

In another implementation form of the first aspect, the mandibular canal segmentation data is obtained as an output of a trained neural network. The method can, for example, postprocess the mandibular canal segmentation data and thus provide the at least one candidate mandibular canal pair with improved accuracy, as compared to the neural network alone.

According to a second aspect, a computing device comprises at least one processor and at least one memory including computer program code, the at least one memory and the computer program code configured to, with the at least one processor, cause the computing device to perform the method according to the first aspect.

According to a third aspect, a computer program product comprises program code configured to perform the method according to the first aspect when the computer program product is executed on a computer.

Many of the attendant features will be more readily appreciated as they become better understood by reference to the following detailed description considered in connection with the accompanying drawings.

### DESCRIPTION OF THE DRAWINGS

In the following, embodiments are described in more detail with reference to the attached figures and drawings, in which:
Fig. 1 illustrates a flow chart representation of a method according to an embodiment;
Fig. 2 illustrates a schematic representation of a computing device according to an embodiment;
Fig. 3 illustrates a schematic representation of a plurality of possible mandibular canal voxels and a corresponding voxel structures according to an embodiment;
Fig. 4 illustrates a schematic representation of a voxel structures and corresponding routes according to an embodiment;
Fig. 5 illustrates a schematic representation of a voxel structures as connected components according to an embodiment;
Fig. 6 illustrates a flow chart representation of a concatenation procedure according to an embodiment;
Fig. 7 illustrates a schematic representation of distances between routes according to an embodiment;
Fig. 8 illustrates a schematic representation of route segments according to an embodiment;
Fig. 9 illustrates a schematic representation of symmetry score calculation according to an embodiment;
Fig. 10 illustrates a flow chart representation of a candidate mandibular canal pair selection procedure according to an embodiment; and
Fig. 11 illustrates a schematic representation of a convolutional neural network according to an embodiment.

In the following, like reference numerals are used to designate like parts in the accompanying drawings.

### DETAILED DESCRIPTION

In the following description, reference is made to the accompanying drawings, which form part of the disclosure, and in which are shown, by way of illustration, specific aspects in which the present disclosure may be placed. It is understood that other aspects may be utilised, and structural or logical changes may be made without departing from the scope of the present disclosure. The following detailed description, therefore, is not to be taken in a limiting sense, as the scope of the present disclosure is defined by the appended claims.

For instance, it is understood that a disclosure in connection with a described method may also hold true for a corresponding device or system configured to perform the method and vice versa. For example, if a specific method step is described, a corresponding device may include a unit to perform the described method step, even if such unit is not explicitly described or illustrated in the figures. On the other hand, for example, if a specific apparatus is described based on functional units, a corresponding method may include a step performing the described functionality, even if such step is not explicitly described or illustrated in the figures. Further, it is understood that the features of the various example aspects described herein may be combined with each other, unless specifically noted otherwise.

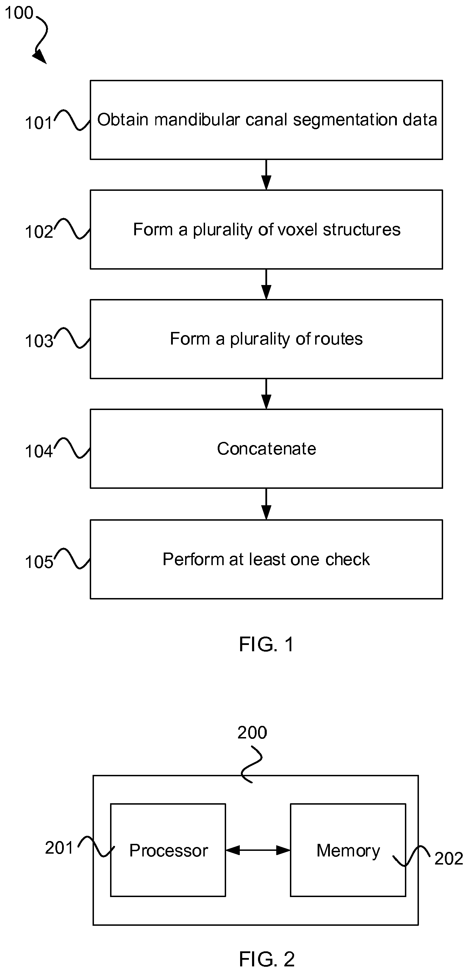

Fig. 1 illustrates a flow chart representation of a method according to an embodiment.

According to an embodiment, the method 100 comprises obtaining 101 mandibular canal segmentation data comprising a plurality of possible mandibular canal voxels in a computerized tomography scan of a mandible, wherein each voxel in the plurality of possible mandibular canal voxels is associated with a probability value quantifying a probability that the voxel comprises a mandibular canal.

The mandible may also be referred to as the lower jaw, jawbone, or similar.

Herein, a voxel may represent a value on a regular grid in three-dimensional space. A voxel represents a sample, or data point, on a regularly spaced three-dimensional grid. A voxel can represent a single point on this grid. The data point can comprise a single piece of data, such as the probability value quantifying the probability that the voxel comprises a mandibular canal, or multiple pieces of data. The probability value may not comprise the probability itself. Rather, the probability value may comprise any quantity that quantifies the probability.

A mandibular canal voxel may refer to a voxel that corresponds to a region of the computerized tomography scan comprising a mandibular canal. Similarly, any voxel in the plurality of possible mandibular canal voxels may correspond to a region of the computerized tomography scan comprising a mandibular canal and the probability associated with each voxel quantifies the probability that the region of the voxel comprises a mandibular canal.

Herein, the geometry of the computerized tomography scan, of the mandible, and/or of the mandibular canals can be described by referring to the depth, width, and height dimensions/directions. The depth dimension/direction may also be referred to as the front-back dimension/direction, z-dimension, or similar. The depth dimension/direction can be perpendicular to the coronal plane. The width dimension/direction may also be referred to as the left-right dimension/direction, x-dimension, or similar. The width dimension/direction can be perpendicular to the sagittal plane. The height dimension/direction may also be referred to as the bottom-up dimension/direction, y-dimension, or similar. The height dimension/direction can be perpendicular to the horizontal/axial/transverse plane. The zero point of the x-axis, of the y-axis, and/or of the z-axis may be placed to be in the middle of the scan of the mandible.

The method 100 may further comprise forming 102 a plurality of voxel structures by connecting neighbouring voxels with a probability value above a preconfigured probability threshold in the possible mandibular canal voxels.

Herein, a voxel structure may comprise any structure comprising a plurality of connected voxels. For example, a voxel structure can be implemented as a connected component as disclosed herein. A voxel structure may comprise a plurality of connected voxels. Thus, each voxel in a voxel structure is neighboured by at least one other voxel in the same voxel structure.

The preconfigured probability threshold may be, for example, 0.1, 0.2, 0.3, 0.4, 0.5, 0.6, 0.7, 0.8, or 0.9.

Small voxel structures may be discarded after the voxel structures have been formed. For example, voxel structure the size (i.e. the number of voxels in the voxel structure) of which is smaller than a preconfigured percentage of the largest voxel structure may be discarded. For example, voxel structures *C* with $size \left(C\right) \geq T \times {max}_{c} size \left(c\right)$ may be kept, where *T* is a threshold value, such as 0.01, 0.02, 0.03, 0.05, or 0.1. A smaller threshold may be chosen in order to include more candidate voxels and thus not to ignore small voxel structures.

The method 100 may further comprise forming 103 a plurality of routes by skeletonizing each voxel structure in the plurality of voxel structures, wherein each route in the plurality of routes comprises a spatial curve of a corresponding voxel structure in the plurality of voxel structures.

Each route in the plurality of routes can be obtained by skeletonizing a corresponding voxel structure in the plurality of voxel structures.

The method 100 may further comprise inspecting 104, for each route pair in the plurality of routes, whether the route pair fulfils a concatenation criterion, and in response to a route pair fulfilling the concatenation criterion, concatenating the route pair, thus forming a second plurality of routes.

Herein, concatenating two routes may comprise creating a new route by joining the two routes end-to-end.

The method 100 may further comprise performing 105 at least one check on at least one spatial feature of each route in the second plurality of routes and choosing at least one candidate mandibular canal pair from the second plurality of routes based on the at least one check.

Mandibular canals are mostly oriented going downwards and inwards. The at least one check on at least one spatial feature of each route in the second plurality of routes can use this and other information about the geometry of mandibular canals to choose at least one candidate mandibular canal pair from the second plurality of routes.

The method 100 may further comprise providing the at least one candidate mandibular canal pair. The providing may comprise, for example, providing the at least one candidate mandibular canal pair to a user. The at least one candidate mandibular canal pair may be provided to a user by, for example, displaying the corresponding routes to a user on a display. The routes may be, for example, overlayed on top of the computerized tomography scan of the mandible. In some embodiments, key points of a route can be samples and the key points can be provided.

At least some embodiments of the method 100 can take into account also small route segments that would be otherwise ignored.

At least some embodiments of the method 100 can consider the shapes of the extracted routes so that other anatomic structures and even mislabelled data can be ignored.

The method 100 can construct a correct segmentation of mandibular nerve canals from incomplete segmentation data that can comprise gaps and false positives. The method 100 can work on both cone-beam computed tomography (CBCT) and computed tomography (CT) data. The method 100 can construct all possible canals which can then be screened based on, for example, route coordinate monotonicity and pairwise symmetricity.

The method 100 can first construct all possible mandibular canals suggested by the mandibular canal segmentation data and then use heuristic methods to screen out the false canals. The method 100 can then use checks to screen out routes/route pairs that are less likely to correspond to mandibular canal routes. Such checks can consider spatial characteristics of mandibular canal routes.

Fig. 2 illustrates a schematic representation of a computing device 200 according to an embodiment.

According to an embodiment, the computing device 200 comprises at least one processor 201 and at least one memory 202 including computer program code.

The at least one memory 202 and the computer program code may be configured to, with the at least one processor 201, cause the computing device 200 to perform the method 100.

The at least one processor 201 may comprise, for example, one or more of various processing devices, such as a central processing unit (CPU), a graphical processing unit (GPU), co-processor, a microprocessor, a processing unit, a digital signal processor (DSP), a processing circuitry with or without an accompanying DSP, or various other processing devices including integrated circuits such as, for example, an application specific integrated circuit (ASIC), a field programmable gate array (FPGA), a microprocessor unit (MCU), a hardware accelerator, such as a neural network accelerator, a special-purpose computer chip, or the like.

The at least one memory 202 may be configured to store, for example, computer programs and the like. The at least one memory 202 may comprise one or more volatile memory devices, one or more non-volatile memory devices, and/or a combination of one or more volatile memory devices and non-volatile memory devices. For example, the at least one memory 202 may be embodied as magnetic storage devices (such as hard disk drives, floppy disks, magnetic tapes, etc.), optical magnetic storage devices, and semiconductor memories (such as mask ROM, PROM (programmable ROM), EPROM (erasable PROM), flash ROM, RAM (random access memory), etc.).

The computing device 200 may further comprise other components not illustrated in the embodiment of Fig. 2. The computing device 200 may comprise, for example, an input/output bus for connecting the computing device 200 to other devices.

When the computing device 200 is configured to implement some functionality, some component and/or components of the computing device 200, such as the at least one processor 201 and/or the at least one memory 202, may be configured to implement this functionality. Furthermore, when the at least one processor 201 is configured to implement some functionality, this functionality may be implemented using program code comprised, for example, in the at least one memory 202.

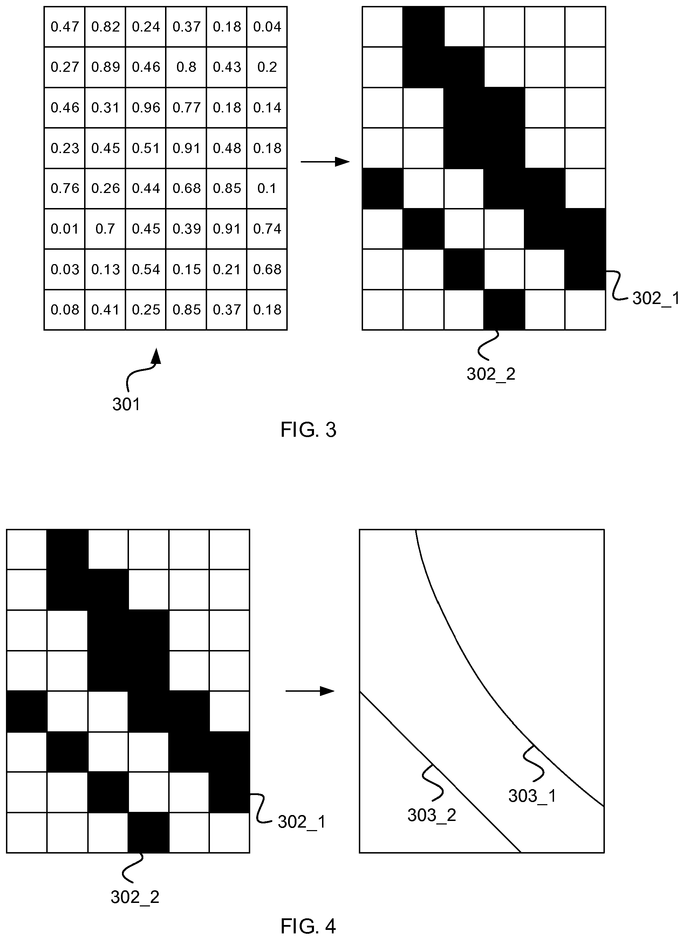

Fig. 3 illustrates a schematic representation of a plurality of possible mandibular canal voxels and a corresponding voxel structures according to an embodiment.

The plurality of voxel structures can be formed by connecting neighbouring voxels with a probability value above a preconfigured probability threshold in the possible mandibular canal voxels. For example, in the embodiment of Fig. 3, the probability threshold is set to 0.5. Thus, voxel structures 302_1, 302_2 illustrated in the embodiment of Fig. 3 are formed by connecting neighbouring voxels in the possible mandibular canal voxels 301 with a probability value above 0.5 into the voxel structures 302_1, 302_2.

The rule used for whether two voxels are neighbouring may differ in different embodiments. For example, in the embodiment of Fig. 3, for each voxel, eight nearest voxels are considered to be neighbouring voxels. If only four nearest voxels would be considered to be neighbouring voxels, the second 302_2 voxel structure would not be formed. In three dimensions, for each voxel, the six other voxels touching the voxel or the 26 other voxels within a 3x3x3 voxel cube may be considered to be neighbouring voxels, for example.

It should be appreciated that the embodiment of Fig. 3 is only a two-dimensional representation of a simplified example that illustrates only two voxel structures 302_1, 302_2.

Fig. 4 illustrates a schematic representation of a voxel structures and corresponding routes according to an embodiment.

Each route may be obtained may skeletonizing a voxel structure. Thus, each route may comprise a skeleton of a corresponding voxel structure.

Herein, a skeleton of a voxel structure may refer to a thin version of that voxel structure that is equidistant to boundaries of the voxel structure. Thus, the route can emphasize geometrical and topological properties of the voxel structure, such as its connectivity, topology, length, direction, and width. Together with the distance of its points to the shape boundary, the skeleton can also serve as a representation of the voxel structure.

In the embodiment of Fig. 4, each route 303_1, 303_2 is obtained by skeletonizing a corresponding voxel structure 302_1, 302_2.

According to an embodiment, the method 100 further comprises, after forming the plurality of routes and before inspecting, for each route pair in the plurality of routes, whether the route pair fulfils a concatenation criterion, sorting voxels in each route in the plurality of routes according to a relative location of each voxel in the route.

For example, two nearest-neighbour graphs for voxels in *R* can be constructed. A first graph G*ₛ* can have a smaller radius for preserving route topology and a second graph *Gₗ* can have a larger radius for neighbour searching. Here, the radius measures which voxels are considered neighbouring to a given voxel.

For example, breadth-first search (BFS) can be used to find a longest path *R̂* in the route *R* based on *Gₛ*. The longest path *R̂* can be used as the sorted route. If the path *R̂* is not long enough (for example if |*R̂*| *< Tᵣ*|*R*|*,* where |·| is the length of the path/route and *Tᵣ* is a threshold value), the longest path *R̂* can be extended by appending nearest neighbouring voxels based on *Gₗ* iteratively until the longest path *R̂* is long enough (for example if |*R̂*| ≥ *Tᵣ*|*R*|). Additional constrains can be used for the nearest neighbouring voxel to be appended. For example, the y-component *v_{y}* of the voxel to be appended may need to greater than the y-component *v̂_{y}* of the last voxel in the longest path *R̂*, wherein the y-axis is along the front-back direction of the computerized tomography scan of the mandible and greater y-values point to the back direction. If such a voxel cannot be found, the constraint can be relaxed to, for example, *v_{y} > v̂_{y} - T_{y},* where *T_{y}* is a threshold value.

Additionally, it can be ensured that the first and the last voxels in the ordered routes are true endpoints by examining their number of neighbouring voxels.

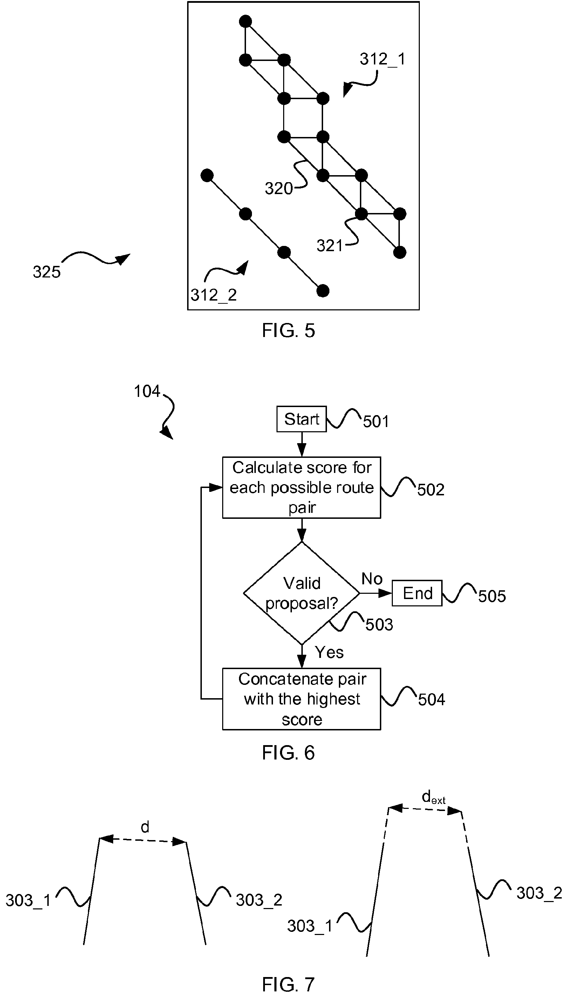

Fig. 5 illustrates a schematic representation of a voxel structures as connected components according to an embodiment.

According to an embodiment, each voxel structure in the plurality of voxel structures is formed using a connected-component algorithm. This may also be referred to as connected-component labelling (CCL) or connected-component analysis (CCA).

A connected-component algorithm can construct a graph 325, comprising vertices 321 and connecting edges 320, based on the plurality of possible mandibular canal voxels 301. Each vertex in the graph 325 can correspond to a voxel. The edges 320 can indicate connected neighbouring voxels. The connected-component algorithm can construct a plurality of connected components 312_1, 312_2. A connected component is a subgraph in which any two vertices 321 are connected to each other by paths, and which is connected to no additional vertices in the rest of the graph 325. Each connected component can correspond to a voxel structure of connected voxels.

The embodiment of Fig. 5 illustrates connected components 312_1, 312_2 corresponding to the voxel structures 302_1 302_2, respectively, illustrated in the embodiments of Fig. 3 and Fig. 4.

Fig. 6 illustrates a flow chart representation of a concatenation procedure according to an embodiment.

The procedure can start at operation 501.

In operation 502, a score for each route pair in the plurality of routes can be calculated. This score may be referred to as concatenation score or similar.

In operation 503, it can be checked whether a valid proposal of a route pair to be concatenated is provided by the score calculation 502. For example, if no route pair fulfils the concatenation criterion, no valid proposal is provided. If no valid proposal is provided, the procedure can move to operation 505 where the procedure ends. If a valid proposal is provided, the procedure can move to operation 504.

According to an embodiment, the concatenation criterion comprises a distance between an endpoint of a first route in the route pair and an endpoint of a second route in the route pair being below a preconfigured maximum threshold distance.

For example, in operation 502, a concatenation score can be calculated for each route pair based on the distance between an endpoint of a first route in the route pair and an endpoint of a second route in the route pair. Additionally or alternatively, other criteria, such as those disclosed herein, may be considered when calculating the concatenation score. Then, in operation 503, it can be checked whether the concatenation score of any route pair is greater than a preconfigured minimum concatenation score, i.e. has the calculation provided a valid proposal of routes to be concatenated. Alternatively or additionally, the score calculation 502 itself may return a special value, such as null, if a valid proposal is not found and this value can be detected in operation 503.

In operation 504, the route pair with the highest score can be concatenated. After the concatenation, the procedure can move back to operation 502, where a score for each route pair can be calculated. Due to the concatenation, performed in operation 504, the plurality of routes now comprises a different plurality of routes. Thus, the operations 502 - 504 can be repeated until no valid concatenation proposal is provided.

As a result of the procedure 104, the second plurality of routes can be provided.

Fig. 7 illustrates a schematic representation of distances between routes according to an embodiment.

According to an embodiment, the inspecting, for each route pair in the plurality of routes, whether the route pair fulfils the concatenation criterion comprises calculating at least one of: a minimum distance between endpoints of the route pair, a minimum distance between extrapolated endpoints of the route pair, wherein the extrapolated endpoints are obtained by linearly extrapolating the endpoints by a preconfigured number of voxels, and/or a skew distance of the route pair.

For each route pair, the minimum of the distances between the endpoints belonging to different routes in the route pair can be calculated. There are 2 × 2 = 4 cases in total. The minimum distance can be denoted as *d*.

The minimum distance between extrapolated endpoints of the route pair can be obtained by, for example, extrapolating the endpoints of each route linearly by *k* voxels. The minimal distance can be calculated and denoted as *dₑₓₜ.*

The skew distance of a route pair may refer to a shortest distance between lines formed by connecting an endpoint of a route to its extrapolation. One purpose of the skew distance is to check whether the two routes will cross when they are extrapolated, while the other two criteria quantify whether two routes are going in opposite directions. The skew distance can be denoted by *d_{skew}.*

The concatenation criterion can comprise the minimum distance between the endpoints fulfilling a first criterion, the minimum distance between the extrapolated endpoints fulfilling a second criterion, and/or the skew distance of the route pair fulfilling a third criterion.

For example, the routes can be concatenated if *dₑₓₜ < T*₁, *d - dₑₓₜ > T*₂, and *d_{skew}* < *T*₃, where *Tᵢ* are preconfigured threshold values. If these criteria are met, *dₑₓₜ* may be provided as the concatenation score, for example in the operation 502 of the embodiment of Fig 6. If these criteria are not met, a special value, such as null, can be provided to indicate this. The route pair to be concatenated can then be chosen based on the concatenation score of each route pair, for example, as disclosed in the embodiment of Fig. 6.

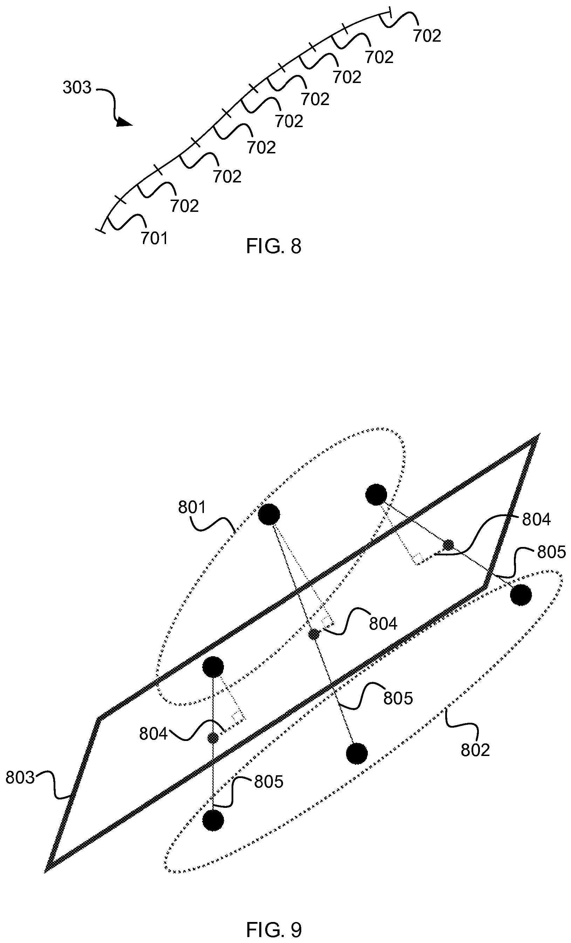

Fig. 8 illustrates a schematic representation of route segments according to an embodiment.

According to an embodiment, the performing of the at least one check on the at least one spatial feature of each route 303 in the second plurality of routes comprises: determining a direction for a first segment 701 of the route 303 and, for each segment 702 in a plurality of segments of the route 303, other than the first segment 701 of the route 303, inspecting whether a direction of the segment 702 is within a preconfigured tolerance from the direction of the first segment 701 of the route 303, calculating a monotonicity score based on a number of segments in the plurality of segments not being within the tolerance, and choosing the at least one candidate mandibular canal pair from the second plurality of routes based on at least the monotonicity score.

The first segments 701 may correspond to any segment in the route 303. For example, in the embodiment of Fig. 8, the first segment 701 is located at one end of the route 303. In other embodiments, the first segment 701 may be located at any location along the route 303.

The plurality of segments may comprise any number of segments. For example, in the embodiment of Fig. 8, the plurality of segments comprises all segments other than the first segment. In other embodiments, the plurality of segments may comprise, for example, only a smaller subset of all segments in the route 303.

For example, near the start of a route 303, it can be checked into which directions *x* (width), *y* (height), and/or *z* (depth) coordinates are going (increasing or decreasing). In some embodiments, only some of these coordinates may be considered. For example, only the *x* and *z* components may be of interest. To get more robustness, some simple voting strategy can be applied here. Then, for every other segment 702, it can be checked whether the route 303 is going along this direction. Some tolerance and some number of violations can be allowed.

Fig. 9 illustrates a schematic representation of symmetry score calculation according to an embodiment.

According to an embodiment, the performing of the at least one check on the at least one spatial feature of each route in the second plurality of routes comprises: for at least one route pair in the second plurality of routes, calculate at least one pair score, wherein the at least one pair score comprises at least one of: a mean coordinate score for the route pair, wherein the mean coordinate score is calculated by comparing a mean coordinate in a coordinate axis direction of each route in the route pair; a symmetry score for the route pair quantifying spatial symmetry of the route pair; and/or a symmetry plane score for the route pair.

A total score for the at least one route pair in the second plurality of routes can be calculated based on the at least one pair score of the pair. The at least one candidate mandibular canal pair from the second plurality of routes can be chosen based on at least the total score.

According to an embodiment, the symmetry score and/or the symmetry plane score is calculated based on digests of the routes in the route pair, wherein the digest of a route comprises a location of a start of the route, a location of an end of the route, and a mean location of the route.

The digest 801 of a first route, the digest 802 of a second route, and a symmetry plane 803 determined based on the digests 801, 802 are illustrated in the embodiment of Fig. 9.

The symmetry score can be calculated based on, for example, an average of displacements 804 of the digests 801, 802 from the symmetry plane 803. Each displacement 804 can quantify how perpendicular a line 805 drawn from a point in the first digest 801 to a corresponding point in the second digest 802 is to the symmetry plane 803. For example, relative placements or sine values of acute angles between the lines 805 and the symmetry plane 803 can be used. When a line 805 is perpendicular to the symmetry plane 803, the displacement may be zero. In other embodiments, the symmetry score can be calculated in a different way.

According to an embodiment, the symmetry plane score quantifies how well a symmetry plane 803 of the route pair is aligned with a height direction of the computerized tomography scan of the mandible. Thus, the symmetry plane score can quantify the left-right symmetry of the route pair.

According to an embodiment, the coordinate axis direction is parallel with a width direction of the computerized tomography scan of the mandible.

Thus, the total score can reflect how well a route pair follows the following observations of a correct pair of mandibular canal routes: the z coordinates of the two routes should have close by mean value, the two routes should be roughly symmetric, the symmetry plane should be along the height (y) direction, and the normal vector of the symmetry plane should be along the width (x) direction.

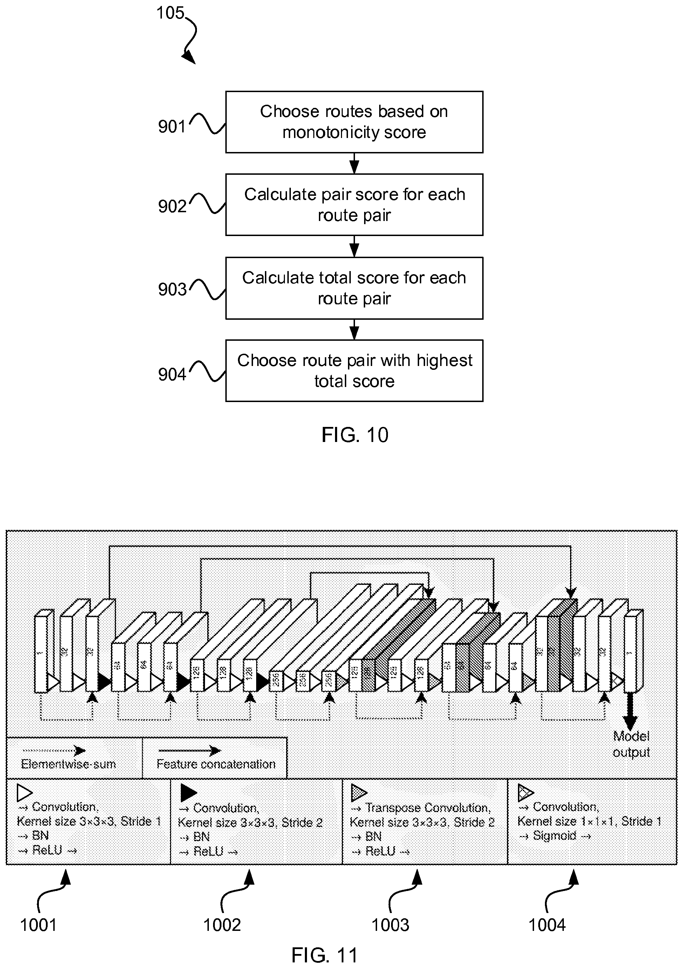

Fig. 10 illustrates a flow chart representation of a candidate mandibular canal pair selection procedure according to an embodiment.

According to an embodiment, the choosing at least one candidate mandibular canal pair from the second plurality of routes based on the at least one check comprises performing at least some of the operations 901 - 904.

In operation 901 a subset of routes from the second plurality of routes can be chosen based on the monotonicity score of each route.

The monotonicity score can be calculated for each route in the second plurality of routes in a manner disclosed herein. The subset can be chosen based by, for example, choosing all routes with a monotonicity score greater than a threshold monotonicity score.

If there are less than two routes left after operation 901, for example less than two routes have a monotonicity score greater than the threshold monotonicity score, some routes whose monotonicity is below the threshold may be chosen for the following operations. Still, routes with the highest scores will be chosen first.

In operation 902, the at least one pair score can be calculated for each route pair in the chosen subset of routes.

The at least one pair score can be calculated for each route pair in the chosen subset of routes in a manner disclosed herein.

In operation 903 the total score can be calculated for each route pair in the chosen subset based on the at least one pair score of the route pair.

The total score can be calculated for each route pair in the chosen subset of routes in a manner disclosed herein.

In operation 904, a route pair with a highest total score in the chosen subset of routes can be chosen as the candidate mandibular canal pair.

Thus, in the embodiment of Fig. 10, the monotonicity score can be used to filter the routes and the candidate mandibular canal pair can then be chosen from the remaining subset of routes based on the total score. Thus, the embodiment of Fig. 10 can choose the at least one candidate mandibular canal pair from the second plurality of routes based on the monotonicity score and the total score.

Fig. 11 illustrates a schematic representation of a convolutional neural network according to an embodiment.

According to an embodiment, the mandibular canal segmentation data is obtained as an output of a trained neural network.

Herein, the term "neural network" is used to refer to an artificial neural network.

In the embodiments of Fig. 11, the neural network comprises a convolutional neural network (CNN). The CNN comprises four types of convolutional layers. A first type 1001 comprises convolution, a convolutional kernel of size 3 × 3 × 3 with a stride of one, batch normalization (BN), and a rectified linear unit (ReLU) activation function. A second type 1002 comprises convolution, a convolutional kernel of size 3 × 3 × 3 with a stride of two, BN, and a ReLU activation function. A third type 1003 comprises transpose convolution, a convolutional kernel of size 3 × 3 × 3 with a stride of two, BN, and a ReLU activation function. A fourth type 1004 comprises convolution, a convolutional kernel of size 1 × 1 × 1 with a stride of one, and a sigmoid activation function. The CNN also comprises skip connections as illustrated in Fig. 11. Some of the skip connections are element-wise sums and some are feature concatenations. The number of channels in each layer is depicted in Fig. 11.

The embodiment of Fig. 11 is only an exemplary implementation of a CNN that can provide the mandibular canal segmentation data. Alternatively, the mandibular canal segmentation data may be provided by any other type of data processing, such as any type of appropriately trained machine learning model or an iterative machine learning model that may not need to be trained with data.

Any range or device value given herein may be extended or altered without losing the effect sought. Also any embodiment may be combined with another embodiment unless explicitly disallowed.

Although the subject matter has been described in language specific to structural features and/or acts, it is to be understood that the subject matter defined in the appended claims is not necessarily limited to the specific features or acts described above. Rather, the specific features and acts described above are disclosed as examples of implementing the claims and other equivalent features and acts are intended to be within the scope of the claims.

It will be understood that the benefits and advantages described above may relate to one embodiment or may relate to several embodiments. The embodiments are not limited to those that solve any or all of the stated problems or those that have any or all of the stated benefits and advantages. It will further be understood that reference to 'an' item may refer to one or more of those items.

The steps of the methods described herein may be carried out in any suitable order, or simultaneously where appropriate.

The term 'comprising' is used herein to mean including the method, blocks or elements identified, but that such blocks or elements do not comprise an exclusive list and a method or apparatus may contain additional blocks or elements.

It will be understood that the above description is given by way of example only and that various modifications may be made by those skilled in the art. The above specification, examples and data provide a complete description of the structure and use of exemplary embodiments. Although various embodiments have been described above with a certain degree of particularity, or with reference to one or more individual embodiments, those skilled in the art could make numerous alterations to the disclosed embodiments without departing from the scope of the invention, which is defined in the appended claims.

## Claims

1. A computer-implemented method for postprocessing of mandibular canal segmentation data, comprising:
- obtaining mandibular canal segmentation data comprising a plurality of possible mandibular canal voxels in a computerized tomography scan of a mandible, wherein each voxel in the plurality of possible mandibular canal voxels is associated with a probability value quantifying a probability that the voxel comprises a mandibular canal;
- forming a plurality of voxel structures by connecting neighbouring voxels with a probability value above a preconfigured probability value threshold in the possible mandibular canal voxels;
- forming a plurality of routes by skeletonizing each voxel structure in the plurality of voxel structures, wherein each route in the plurality of routes comprises a spatial curve of a corresponding voxel structure in the plurality of voxel structures;
- inspecting, for each route pair in the plurality of routes, whether the route pair fulfils a concatenation criterion, and in response to a route pair fulfilling the concatenation criterion, concatenating the route pair, thus forming a second plurality of routes; and
- performing at least one check on at least one spatial feature of each route in the second plurality of routes and choosing at least one candidate mandibular canal pair from the second plurality of routes based on the at least one check.

2. The method according to claim 1, further comprising, after forming the plurality of routes and before inspecting for each route pair in the plurality of routes whether the route pair fulfils a concatenation criterion, sorting voxels in each route in the plurality of routes according to a relative location of each voxel in the route.

3. The method according to claim 1 or claim 2, wherein the performing of the at least one check on the at least one spatial feature of each route in the second plurality of routes and choosing at least one candidate mandibular canal pair from the second plurality of routes based on the at least one check comprises:
- determining a direction for a first segment of the route;
- for each segment in a plurality of segments of the route, other than the first segment of the route, inspecting whether a direction of the segment is within a preconfigured tolerance from the direction of the first segment of the route;
- calculating a monotonicity score based on a number of segments in the plurality of segments not being within the tolerance; and
- choosing the at least one candidate mandibular canal pair from the second plurality of routes based on at least the monotonicity score.

4. The method according to any preceding claim, wherein the performing of the at least one check on the at least one spatial feature of each route in the second plurality of routes and choosing at least one candidate mandibular canal pair from the second plurality of routes based on the at least one check comprises:
- for at least one route pair in the second plurality of routes, calculating at least one pair score, wherein the at least one pair score comprises at least one of:
- a mean coordinate score for the route pair, wherein the mean coordinate score is calculated by comparing a mean coordinate in a coordinate axis direction of each route in the route pair,
- a symmetry score for the route pair quantifying spatial symmetry of the route pair, and/or
- a symmetry plane score for the route pair;
- calculating a total score for the at least one route pair in the second plurality of routes based on the at least one pair score of the pair; and
- choosing the at least one candidate mandibular canal pair from the second plurality of routes based on at least the total score.

5. The method according to claim 4, wherein the performing of the at least one check on the at least one spatial feature of each route in the second plurality of routes and choosing at least one candidate mandibular canal pair from the second plurality of routes based on the at least one check comprises:
- choosing a subset of routes from the second plurality of routes based on the monotonicity score of each route;
- calculating the at least one pair score for each route pair in the chosen subset of routes;
- calculating the total score for each route pair in the chosen subset based on the at least one pair score of the route pair; and
- choosing a route pair with a highest total score in the chosen subset of routes as the candidate mandibular canal pair.

6. The method according to claim 4 or claim 5, wherein the coordinate axis direction is parallel with a width direction of the computerized tomography scan of the mandible.

7. The method according to any of claims claim 4 - 6, wherein the symmetry score and/or the symmetry plane score is calculated based on digests of the routes in the route pair, wherein the digest of a route comprises a location of a start of the route, a location of an end of the route, and a mean location of the route.

8. The method according to any of claims 4 - 7, wherein the symmetry plane score quantifies how well a symmetry plane of the route pair is aligned with a height direction of the computerized tomography scan of the mandible.

9. The method according to any preceding claim, wherein the concatenation criterion comprises a distance between an endpoint of a first route in the route pair and an endpoint of a second route in the route pair being below a preconfigured maximum threshold distance.

10. The method according to any preceding claim, wherein the inspecting, for each route pair in the plurality of routes, whether the route pair fulfils the concatenation criterion comprises calculating at least one of:
- a minimum distance between endpoints of the route pair,
- a minimum distance between extrapolated endpoints of the route pair, wherein the extrapolated endpoints are obtained by linearly extrapolating the endpoints by a preconfigured number of voxels, and/or
- a skew distance of the route pair,
- wherein the concatenation criterion comprises the minimum distance between the endpoints fulfilling a first criterion, the minimum distance between the extrapolated endpoints fulfilling a second criterion, and/or the skew distance of the route pair fulfilling a third criterion.

11. The method according to any preceding claim, wherein each voxel structure in the plurality of voxel structures is formed using a connected-component algorithm.

12. The method according to any preceding claim, wherein the mandibular canal segmentation data is obtained as an output of a trained neural network.

13. A computing device, comprising:
- at least one processor; and
- at least one memory including computer program code;
- the at least one memory and the computer program code configured to, with the at least one processor, cause the computing device to perform the method according to any of claims 1 - 12.

14. A computer program product comprising program code configured to perform the method according to any of claims 1 - 12 when the computer program product is executed on a computer.

## Patentansprüche

1. Computerimplementiertes Verfahren zur Nachverarbeitung von Unterkieferkanal-Segmentierungsdaten, mit:
- Erhalten von Unterkieferkanal-Segmentierungsdaten, die eine Vielzahl von möglichen Unterkieferkanal-Voxeln in einem computergestützten Tomographiescan eines Unterkiefers umfassen, wobei jedes Voxel in der Vielzahl von möglichen Unterkieferkanal-Voxeln mit einem Wahrscheinlichkeitswert assoziiert ist, der eine Wahrscheinlichkeit quantifiziert, dass das Voxel einen Unterkieferkanal umfasst;
- Bilden einer Vielzahl von Voxelstrukturen durch Verbinden benachbarter Voxel mit einem Wahrscheinlichkeitswert über einem vorkonfigurierten Wahrscheinlichkeitswert-Schwellenwert in den möglichen Unterkieferkanal-Voxeln;
- Bilden einer Vielzahl von Routen durch Skelettieren jeder Voxelstruktur in der Vielzahl von Voxelstrukturen, wobei jede Route in der Vielzahl von Routen eine räumliche Kurve einer entsprechenden Voxelstruktur in der Vielzahl von Voxelstrukturen umfasst;
- Überprüfen, für jedes Routenpaar in der Vielzahl von Routen, ob das Routenpaar ein Verkettungskriterium erfüllt, und als Reaktion darauf, dass ein Routenpaar das Verkettungskriterium erfüllt, Verketten des Routenpaars, wodurch eine zweite Vielzahl von Routen gebildet wird; und
- Durchführen mindestens einer Prüfung an mindestens einem räumlichen Merkmal jeder Route in der zweiten Vielzahl von Routen und Auswählen mindestens eines Kandidaten-Unterkieferkanalpaars aus der zweiten Vielzahl von Routen basierend auf der mindestens einen Prüfung.

2. Verfahren nach Anspruch 1, ferner mit, nach dem Bilden der Vielzahl von Routen und vor dem Überprüfen für jedes Routenpaar in der Vielzahl von Routen, ob das Routenpaar ein Verkettungskriterium erfüllt, Sortieren von Voxeln in jeder Route in der Vielzahl von Routen gemäß einer relativen Position jedes Voxels in der Route.

3. Verfahren nach Anspruch 1 oder Anspruch 2, wobei das Durchführen der mindestens einen Prüfung an dem mindestens einen räumlichen Merkmal jeder Route in der zweiten Vielzahl von Routen und das Auswählen mindestens eines Kandidaten-Unterkieferkanalpaars aus der zweiten Vielzahl von Routen basierend auf der mindestens einen Prüfung Folgendes umfasst:
- Bestimmen einer Richtung für ein erstes Segment der Route;
- für jedes Segment in einer Vielzahl von Segmenten der Route, das sich von dem ersten Segment der Route unterscheidet, Überprüfen, ob eine Richtung des Segments innerhalb einer vorkonfigurierten Toleranz von der Richtung des ersten Segments der Route liegt;
- Berechnen eines Monotoniewerts basierend auf einer Anzahl von Segmenten in der Vielzahl von Segmenten, die nicht innerhalb der Toleranz liegen; und
- Auswählen des mindestens einen Kandidaten-Unterkieferkanalpaars aus der zweiten Vielzahl von Routen basierend auf mindestens dem Monotoniewert.

4. Verfahren nach einem der vorhergehenden Ansprüche, wobei das Durchführen der mindestens einen Prüfung an dem mindestens einen räumlichen Merkmal jeder Route in der zweiten Vielzahl von Routen und das Auswählen mindestens eines Kandidaten-Unterkieferkanalpaars aus der zweiten Vielzahl von Routen basierend auf der mindestens einen Prüfung Folgendes umfasst:
- für mindestens ein Routenpaar in der zweiten Vielzahl von Routen, Berechnen mindestens eines Paarwerts, wobei der mindestens eine Paarwert mindestens eines umfasst aus der Gruppe von:
- einem mittleren Koordinatenwert für das Routenpaar, wobei der mittlere Koordinatenwert durch Vergleichen einer mittleren Koordinate in einer Koordinatenachsenrichtung jeder Route in dem Routenpaar berechnet wird,
- einem Symmetriewert für das Routenpaar, der die räumliche Symmetrie des Routenpaars quantifiziert, und/oder
- einem Symmetrieebenen-Wert für das Routenpaar;
- Berechnen eines Gesamtwerts für das mindestens eine Routenpaar in der zweiten Vielzahl von Routen basierend auf dem mindestens einen Paarwert des Paars; und
- Auswählen des mindestens einen Kandidaten-Unterkieferkanalpaars aus der zweiten Vielzahl von Routen basierend auf mindestens dem Gesamtwert.

5. Verfahren nach Anspruch 4, wobei das Durchführen der mindestens einen Prüfung an dem mindestens einen räumlichen Merkmal jeder Route in der zweiten Vielzahl von Routen und das Auswählen mindestens eines Kandidaten-Unterkieferkanalpaars aus der zweiten Vielzahl von Routen basierend auf der mindestens einen Prüfung Folgendes umfasst:
- Auswählen einer Teilmenge von Routen aus der zweiten Vielzahl von Routen basierend auf dem Monotoniewert jeder Route;
- Berechnen des mindestens einen Paarwerts für jedes Routenpaar in der ausgewählten Teilmenge von Routen;
- Berechnen des Gesamtwerts für jedes Routenpaar in der ausgewählten Teilmenge basierend auf dem mindestens einen Paarwert des Routenpaars; und
- Auswählen eines Routenpaars mit einem höchsten Gesamtwert in der ausgewählten Teilmenge von Routen als das Kandidaten-Unterkieferkanalpaar.

6. Verfahren nach Anspruch 4 oder Anspruch 5, wobei die Koordinatenachsenrichtung parallel zu einer Breitenrichtung des computergestützten Tomographiescans des Unterkiefers ist.

7. Verfahren nach einem der Ansprüche 4 bis 6, wobei der Symmetriewert und/oder der Symmetrieebenen-Wert basierend auf Zusammenfassungen der Routen in dem Routenpaar berechnet wird, wobei die Zusammenfassung einer Route eine Position eines Starts der Route, eine Position eines Endes der Route und eine mittlere Position der Route umfasst.

8. Verfahren nach einem der Ansprüche 4 bis 7, wobei der Symmetrieebenen-Wert quantifiziert, wie gut eine Symmetrieebene des Routenpaars mit einer Höhenrichtung des computergestützten Tomographiescans des Unterkiefers ausgerichtet ist.

9. Verfahren nach einem der vorhergehenden Ansprüche, wobei das Verkettungskriterium umfasst, dass ein Abstand zwischen einem Endpunkt einer ersten Route in dem Routenpaar und einem Endpunkt einer zweiten Route in dem Routenpaar unter einem vorkonfigurierten maximalen Schwellenwertabstand liegt.

10. Verfahren nach einem der vorhergehenden Ansprüche, wobei das Überprüfen, für jedes Routenpaar in der Vielzahl von Routen, ob das Routenpaar das Verkettungskriterium erfüllt, das Berechnen mindestens eines umfasst aus der Gruppe von:
- einem Mindestabstand zwischen Endpunkten des Routenpaars,
- einem Mindestabstand zwischen extrapolierten Endpunkten des Routenpaars, wobei die extrapolierten Endpunkte durch lineares Extrapolieren der Endpunkte um eine vorkonfigurierte Anzahl von Voxeln erhalten werden, und/oder
- einem Versatzabstand des Routenpaars,
- wobei das Verkettungskriterium umfasst, dass der Mindestabstand zwischen den Endpunkten ein erstes Kriterium erfüllt, der Mindestabstand zwischen den extrapolierten Endpunkten ein zweites Kriterium erfüllt und/oder der Versatzabstand des Routenpaars ein drittes Kriterium erfüllt.

11. Verfahren nach einem der vorhergehenden Ansprüche, wobei jede Voxelstruktur in der Vielzahl von Voxelstrukturen unter Verwendung eines Algorithmus mit verbundenen Komponenten gebildet wird.

12. Verfahren nach einem der vorhergehenden Ansprüche, wobei die Unterkieferkanal-Segmentierungsdaten als eine Ausgabe eines trainierten neuronalen Netzwerks erhalten werden.

13. Rechenvorrichtung, mit:
- mindestens einem Prozessor; und
- mindestens einem Speicher, der Computerprogrammcode beinhaltet;
- wobei der mindestens eine Speicher und der Computerprogrammcode konfiguriert sind, um mit dem mindestens einen Prozessor die Rechenvorrichtung zu veranlassen, das Verfahren nach einem der Ansprüche 1 bis 12 durchzuführen.

14. Computerprogrammprodukt, mit Programmcode, der konfiguriert ist, um das Verfahren nach einem der Ansprüche 1 bis 12 durchzuführen, wenn das Computerprogrammprodukt auf einem Computer ausgeführt wird.

## Revendications

1. Procédé mis en œuvre par ordinateur de post-traitement de données de segmentation du canal mandibulaire, comprenant :
- l'obtention de données de segmentation du canal mandibulaire comprenant une pluralité de voxels possibles du canal mandibulaire dans un balayage de tomographie par ordinateur d'une mandibule, dans lequel chaque voxel de la pluralité de voxels possibles du canal mandibulaire est associé à une valeur de probabilité quantifiant la probabilité que le voxel comprenne un canal mandibulaire ;
- la formation d'une pluralité de structures de voxel en connectant des voxels voisins avec une valeur de probabilité supérieure à un seuil de valeur de probabilité préconfiguré dans les voxels possibles du canal mandibulaire ;
- la formation d'une pluralité d'itinéraires en squelettisant chaque structure de voxel dans la pluralité de structures de voxel, dans lequel chaque itinéraire dans la pluralité d'itinéraires comprend une courbe spatiale d'une structure de voxel correspondante dans la pluralité de structures de voxel ;
- l'inspection, pour chaque paire d'itinéraires dans la pluralité d'itinéraires, pour savoir si la paire d'itinéraires remplit un critère de concaténation, et en réponse à une paire d'itinéraires remplissant le critère de concaténation, la concaténation de la paire d'itinéraires, ce qui permet de former une seconde pluralité d'itinéraires ; et
- la réalisation d'au moins un contrôle sur au moins une caractéristique spatiale de chaque itinéraire dans la seconde pluralité d'itinéraires et le choix d'au moins une paire de canaux mandibulaires candidats parmi la seconde pluralité d'itinéraires sur la base du au moins un contrôle.

2. Procédé selon la revendication 1, comprenant en outre, après la formation de la pluralité d'itinéraires et avant l'inspection pour chaque paire d'itinéraires dans la pluralité d'itinéraires pour savoir si la paire d'itinéraires remplit un critère de concaténation, le tri de voxels dans chaque itinéraire dans la pluralité d'itinéraires selon un emplacement relatif de chaque voxel dans l'itinéraire.

3. Procédé selon la revendication 1 ou la revendication 2, dans lequel la réalisation de la au moins une vérification sur la au moins une caractéristique spatiale de chaque itinéraire dans la seconde pluralité d'itinéraires et le choix d'au moins une paire de canaux mandibulaires candidats de la seconde pluralité d'itinéraires sur la base du au moins un contrôle, comprend :
- la détermination d'une direction pour un premier segment de l'itinéraire ;
- pour chaque segment dans une pluralité de segments de l'itinéraire, autre que le premier segment de l'itinéraire, l'inspection pour savoir si une direction du segment se trouve dans une tolérance préconfigurée par rapport à la direction du premier segment de l'itinéraire ;
- le calcul d'un score de monotonie sur la base du nombre de segments dans la pluralité de segments qui ne se trouvent pas dans la tolérance ; et
- le choix de la au moins une paire de canaux mandibulaires candidats parmi la seconde pluralité de voies sur la base au moins du score de monotonie.

4. Procédé selon une quelconque revendication précédente, dans lequel la réalisation du au moins un contrôle sur la au moins une caractéristique spatiale de chaque itinéraire dans la seconde pluralité d'itinéraires et le choix d'au moins une paire de canaux mandibulaires candidats parmi la seconde pluralité d'itinéraires sur la base du au moins un contrôle, comprend :
- pour au moins une paire d'itinéraires dans la seconde pluralité d'itinéraires, le calcul d'u moins un score de paire, dans lequel le au moins un score de paire comprend au moins un :
- d'un score de coordonnées moyen pour la paire d'itinéraires, dans lequel le score de coordonnées moyen est calculé en comparant une coordonnée moyenne dans une direction d'axe de coordonnées de chaque itinéraire de la paire d'itinéraires,
- d'un score de symétrie pour la paire d'itinéraires quantifiant une symétrie spatiale de la paire d'itinéraires, et/ou
- d'un score de plan de symétrie pour la paire de routes ;
- le calcul d'un score total pour la au moins une paire d'itinéraires dans la seconde pluralité d'itinéraires sur la base du au moins un score de paire de la paire ; et
- le choix de la au moins une paire de canaux mandibulaires candidats parmi la seconde pluralité de voies sur la base au moins du score total.

5. Procédé selon la revendication 4, dans lequel la réalisation du au moins un contrôle sur la au moins une caractéristique spatiale de chaque itinéraire de la seconde pluralité d'itinéraires et le choix d'au moins une paire de canaux mandibulaires candidats parmi la seconde pluralité d'itinéraires sur la base du au moins un contrôle comprennent :
- le choix d'un sous-ensemble d'itinéraires parmi la seconde pluralité d'itinéraires sur la base du score de monotonie de chaque itinéraire ;
- le calcul du ai moins un score de paire pour chaque paire d'itinéraires dans le sous-ensemble choisi d'itinéraires ;
- le calcul du score total pour chaque paire d'itinéraires dans le sous-ensemble choisi, sur la base du au moins un score de paire de la paire d'itinéraires ; et
- le choix d'une paire d'itinéraires ayant le score total le plus élevé dans le sous-ensemble choisi d'itinéraires comme paire de canaux mandibulaires candidats.

6. Procédé selon la revendication 4 ou la revendication 5, dans lequel la direction d'axe de coordonnées est parallèle au sens de la largeur du balayage de tomographie par ordinateur de la mandibule.

7. Procédé selon l'une quelconque des revendications 4-6, dans lequel le score de symétrie et/ou le score de plan de symétrie sont calculés sur la base de condensés des itinéraires dans la paire d'itinéraires, dans lequel le condensé d'un itinéraire comprend un emplacement de début de l'itinéraire, un emplacement de fin de l'itinéraire et un emplacement moyen de l'itinéraire.

8. Procédé selon l'une quelconque des revendications 4-7, dans lequel le score de plan de symétrie quantifie la manière dont un plan de symétrie de la paire d'itinéraires est aligné sur le sens de la hauteur du balayage de tomographie par ordinateur de la mandibule.

9. Procédé selon une quelconque revendication précédente, dans lequel le critère de concaténation comprend une distance entre un point d'extrémité d'un premier itinéraire dans la paire d'itinéraires et un point d'extrémité d'un second itinéraire dans la paire d'itinéraires étant inférieure à une distance de seuil maximale préconfigurée.

10. Procédé selon une quelconque revendication précédente, dans lequel l'inspection, pour chaque paire d'itinéraires parmi la pluralité d'itinéraires, pour savoir si la paire d'itinéraires remplit le critère de concaténation comprend le calcul d'au moins une :
- d'une distance minimale entre des points d'extrémité de la paire d'itinéraires,
- d'une distance minimale entre des points d'extrémité extrapolés de la paire d'itinéraires, dans lequel les points d'extrémité extrapolés sont obtenus en extrapolant linéairement les points d'extrémité par un nombre préconfiguré de voxels, et/ou
- d'une distance oblique de la paire d'itinéraires,
- dans lequel le critère de concaténation comprend la distance minimale entre les points d'extrémité satisfaisant un premier critère, la distance minimale entre les points d'extrémité extrapolés satisfaisant un deuxième critère, et/ou la distance oblique de la paire d'itinéraires remplissant un troisième critère.

11. Procédé selon une quelconque revendication précédente, dans lequel chaque structure de voxel de la pluralité de structures de voxel est formée à l'aide d'un algorithme de composantes connexes.

12. Procédé selon une quelconque revendication précédente, dans lequel les données de segmentation du canal mandibulaire sont obtenues en sortie d'un réseau neuronal entraîné.

13. Dispositif informatique, comprenant :
- au moins un processeur ; et
- au moins une mémoire incluant un code de programme informatique ;
- la au moins une mémoire et le code du programme informatique étant configurés, avec le au moins un processeur, pour amener le dispositif informatique à réaliser le procédé selon l'une quelconque des revendications 1-12.

14. Produit de programme d'ordinateur comprenant un code de programme configuré pour réaliser le processeur le procédé selon l'une quelconque des revendications 1-12 lorsque le produit de programme informatique est exécuté sur un ordinateur.
